(19) Europäisches Patentamt / European Patent Office / Office européen des brevets

(11) **EP 4 198 119 A1**

(12) **EUROPEAN PATENT APPLICATION**

(43) Date of publication:
**21.06.2023 Bulletin 2023/25**

(21) Application number: **21215336.5**

(22) Date of filing: **16.12.2021**

(51) International Patent Classification (IPC):
***C12M 3/06*** *(2006.01)*

(52) Cooperative Patent Classification (CPC):
**C12M 23/16**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Institute of Solid State Physics, University of Latvia**
**1063 Riga (LV)**

(72) Inventors:
• **MOZOLEVSKIS, Gatis**
  **LV-1009 Riga (LV)**
• **ABOLS, Arturs**
  **LV-1009 Riga (LV)**
• **RIMSA, Roberts**
  **LV-2101 Babite (LV)**

(74) Representative: **Aera A/S**
  **Niels Hemmingsens Gade 10, 5th Floor**
  **1153 Copenhagen K (DK)**

(54) **A MICROFLUIDIC DEVICE FOR SIMULATING ORGAN FUNCTIONS**

(57)    Disclosed is a microfluidic device for simulating organ functions. The microfluidic device comprising a top plate, a bottom plate and a membrane. The top plate comprising a top channel arranged on a first surface of the top plate. The bottom plate comprising a bottom channel arranged on a first surface of the bottom plate, wherein the top channel is overlapping the bottom channel over an entire length of the top channel. The first surface of the top plate and the first surface of the bottom plate are facing each other. The membrane being arranged between the top plate and the bottom plate so that the top channel and the bottom channel are separated by the membrane. The top plate comprises a top channel inlet port being in fluid communication with the top channel. The top plate comprises a first bottom channel inlet port and a second bottom channel inlet port both being in fluid communication with the bottom channel, wherein the second bottom channel inlet port is arranged further from a longitudinal center of the bottom channel than the first bottom channel inlet port.

Fig. 3

EP 4 198 119 A1

## Description

The present disclosure pertains generally to the field of microfluidic devices. The present disclosure relates generally to a microfluidic device for simulating organ functions, such as an Organ-on-Chip (OOC) device or a Gut-on-Chip (GUC) device.

## BACKGROUND

Animal models are commonly used to study human diseases and treatments. However, these models are often limited in their ability to mimic human conditions, in particular, on molecular and/or cellular levels. As per a 2019 European Union (EU) report on animal use in scientific purposes, in the EU alone in 2017 9.58M animals were used in research and testing. For the United States of America (U.S.) these numbers potentially are almost an order of magnitude higher. The EU has called for a ban on animal testing within the next 30 years.

Organ-on-chip (OOC) devices is a realistic alternative to animal studies, which are already investigated by the EU and the U.S. Food and Drug Administration (FDA), since OOC devices can replicate human tissue or even organ functions, for example intestines for oral drug intake or even microbiome research.Although animal models have been used to analyse host-microbiome interactions and their contributions to pathophysiology, there are no in vitro systems available to verify these interactions in human cells cultured with a complex human microbiome in anaerobic conditions. Thus, there is a great need for experimental models that can sustain complex populations of human aerobic, facultative anaerobic and anaerobic microbiota in contact with living human tissues to analyse dynamic and physiologically relevant human host-microbiome interactions.

Current OOC devices do not create a fully representative model system. As a result of material choice the current devices may suffer from small molecule absorption, high oxygen diffusion, and/or water vapor diffusion through the material, which may result in non-physiologically representative dissolved gas concentrations.

## SUMMARY

Accordingly, there is a need for a microfluidic device, which mitigates, alleviates or addresses the shortcomings existing and provides a microfluidic device which provides a more representative model of a human organ.

Disclosed is a microfluidic device for simulating organ functions. The microfluidic device comprising a top plate, a bottom plate and a membrane. The top plate comprising a top channel arranged on a first surface of the top plate. The bottom plate comprising a bottom channel arranged on a first surface of the bottom plate, wherein the top channel is overlapping the bottom channel over an entire length of the top channel. The first surface of the top plate and the first surface of the bottom plate are facing each other. The membrane being arranged between the top plate and the bottom plate so that the top channel and the bottom channel are separated by the membrane. The top plate comprises a top channel inlet port being in fluid communication with the top channel. The top plate comprises a first bottom channel inlet port and a second bottom channel inlet port both being in fluid communication with the bottom channel, wherein the second bottom channel inlet port is arranged further from a longitudinal center of the bottom channel than the first bottom channel inlet port.

It is an advantage of the present disclosure that the microfluidic device reduces the area that is not overlapping between the top and bottom channels by providing a dedicated bottom inlet port for cell seeding of the bottom channel. By providing the bottom channel with a dedicated bottom channel inlet port for cell seeding, such as a cell seeding port, and placing the dedicated bottom channel inlet port close to, such as adjacent to, the top channel inlet port the length of the bottom channel inlet may be reduced. Thereby, a cell barrier can be formed mainly on the membrane between the top and bottom channels, where the cells may proliferate and form a confluent tissue cell layer, such as a villi layer, on both sides of the membrane which can mimic an organ tissue. By reducing a length of a channel that connects the bottom channel inlet port to the bottom channel, the number of cells that in previous microfluidic devices would be wasted in the parts of the bottom channel that did not overlap with the top channels can be reduced. In previous microfluidic devices, cells are typically seeded through a common inlet port for both cell seeding and fluid flow being arranged at an angle (such as not perpendicular) to a longitudinal axis of the bottom channel, thereby creating an excessively long inlet channel, in which inlet channels some cells would get stuck before they could reach the membrane. By increasing the percentage of overlap between the top channel and the bottom channel, more of the studied signal, such as various studied protein expression levels, are from the cells that are seeded in relevant conditions, such as in areas of the microfluidic device that have epithelial and endothelial cells in contact via the membrane and subsequently experience biological barrier properties. Biological barrier properties include, but are not limited to regulating the passage of ions, solutes, other cells, molecular and cell particle such as extracellular vesicle between two biological compartments such as endothelial and epithelial environment. These biological barrier functions are essential for the development and maintenance of tissues of organs and multicellular organisms overall.

## BRIEF DESCRIPTION OF THE DRAWINGS

The above and other features and advantages of the present disclosure will become readily apparent to those skilled in the art by the following detailed description of exemplary embodiments thereof with reference to the attached drawings, in which:

Fig. 1 illustrates an exploded view of an example microfluidic device according to this disclosure,

Fig. 2 is a perspective view of a first surface of the top plate of the microfluidic device according to this disclosure,

Fig. 3 illustrates a perspective view of an assembled example microfluidic device according to the current disclosure,

Fig. 4 is a perspective view of a cross-section of an example microfluidic device according to this disclosure,

Fig. 5 is a perspective view of a cross-section of an assembled example microfluidic device according to this disclosure,

Fig. 6 illustrates an example connector for interconnecting a plurality of top and/or bottom channels according to this disclosure,

Fig. 7 illustrates an example connector for interconnecting a plurality of top and/or bottom channels according to this disclosure, and

Fig. 8 is a cross-sectional view of the example connector illustrated in Fig. 7.

DETAILED DESCRIPTION

Existing in vitro models, such as Transwell inserts, have been used to study human hostmicrobe interactions. However, such studies can only be carried out over a period of hours before bacterial overgrowth leads to cell injury and/or cell death. More advanced models, such as organoid cultures, have shown great promise for studying host-microbiome interactions, but they are limited in providing a vascular interface and oxygen gradients with below 5% luminal oxygen levels which may be required for co-culture of certain strict anaerobes.[1]

Human intestinal epithelial cells may be grown in a microfluidic culture device separated by a nanoporous membrane from a single facultative anaerobic bacterium (such as Lactobacillus rhamnosus GG) and an obligate anaerobe (such as Bacteroides caccae) cultured under anaerobic conditions in a parallel channel. This allows for analysis of the effects of soluble mediators, but not for analysis of the impact of direct contact between host cells and a complex community of commensal microbes. A 2-channel, microfluidic, human gut OOC device has been previously described as being lined by human Caco-2 intestinal epithelial cells culture under dynamic fluid flow and peristalsis-like mechanical deformations, which enabled establishment of stable co-cultures of a human villi structure of intestinal epithelium in direct contact with up to eight different strains of human commensal gut microbes for weeks in vitro under oxygenated conditions. The living intestinal microbiome however, contains hundreds of different types of bacteria that may be anaerobes and/or facultative aerobes.[2] Villi structures may be seen as small, fingerlike projections built from cells that extend into a lumen, such as into an inside space, of the intestine.

OOC devices are based on the concept that it is possible to replicate certain functions of a human organ by culturing the relevant human organ cells (e.g., gut epithelial cells and endothelium cells for simulating intestine functions, such as gut functions) in horizontal microfluidic channels separated by a porous membrane. The culture media may be flowed over both sides of the membrane, thereby ensuring that the cells are supplied with culture medium and that metabolic waste is removed, which is critical for correct tissue architecture developments, such as villi structures.[3-5]

As the cells proliferate and form a confluent cell layer on both sides of the membrane, an OOC system can be formed that can mimic an organ tissue response to external stimuli. However, to mimic the cell function closely it is necessary to apply relevant shear and tensile stresses to the cells, which would be exerted on the cells in a living system. Application of external forces, in the form of shear stress from fluid flow and mechanical stress in the form of peristaltic membrane stretching, can result in more accurate cell function replication in OOC systems in comparison to current in vitro model systems, such as Petri dishes, Transwell plates, organoids and ex vivo.[6]

Furthermore, healthy intestinal function is often disrupted by human conditions, including disorders and diseases involving different types of bacteria found in the intestines. There is a need for a better platform to test therapeutic compounds related to bacteria associated with intestinal function.

Human gut has an aerobic condition throughout the system. The oxygen content varies throughout the human intestine. Typically, the oxygen range of the system is in the range between 0-10 %. Certain areas of the human intestine however have oxygen levels under 1 %, which is not possible to achieve in the current state of the art devices. According to literature the best known OOC devices have around 2% oxygen content at an inlet of a channel and <1% of oxygen content in an outlet of a channel - where the difference in oxygen content is consumed by cells. This may be disadvantageous because it creates an oxygen gradient across the system, alongside the fact that 2% oxygen level is above the physiologically relevant levels in some parts of human intestine. Furthermore, to create the anaerobic conditions in the epithelial channels, it is necessary to place the entire chip assembly in an anaerobic culture chamber instead of only the liquid. This complicates the experimental setup, makes it expensive and provides a small throughput. Additionally current solutions can not provide different gas concentrations in each channel representing gut lumen and vascularization as this is in vivo, therefore lacking some critical characteristics such as gas exchange between epithelial and endothelial channel.

Furthermore, current state of the art OOC devices, typically, only have a single channel with a fixed design. However, the human intestine is complex and contains 10 different parts (duodenum, jejunum, ileum, appendix, cecum, ascending colon, transverse colon, descending colon, sigmoid colon, rectum), all of which have different surface areas, oxygen concentration, microbiota composition and concentration. Therefore, even connecting currently existing chips in series would not produce a physiologically relevant human gut model. Utilization of a single chip design reduces the cost per part for the gut-on chip manufacturers, but limits utilization of multiple, connected chips to study human intestine models more accurately, where each chip may represent a separate region of the human intestine.

Furthermore, in current state of the art OOC devices, there are large areas of the channels, where top and bottom channels are not overlapping, which means that a large number of cells do not experience barrier-like properties, and therefore proper gas, nutrient and molecular signal exchange does not happen between epithelial and endothelial cells like in vivo. The barrier-like properties include, but are not limited to, a regulation of the passage of ions, solutes, molecular and cell particles, such as extracellular vesicle, between the top channel and the bottom channel, such as between an endothelial and an epithelial environment. These biological barrier functions are essential for the development and maintenance of tissues of organs and multicellular organisms. Furthermore, human gut does not consist of only one type of cells. Human gut consists of multiple parts, which are linked together, such as duodenum, jejunum, appendix, cecum, colon, and rectum. However, current chip designs do not allow to address all of these parts of the organ in a single device.

The current disclosure provides a microfluidic device for simulating organ functions, such as an OOC device, which mitigates, alleviates or addresses the shortcomings existing and provides a microfluidic device which provides a more representative model of a human organ. The microfluidic device according to the current disclosure is a vertically stacked microfluidic device that combines two microfluidic channels separated by a membrane, such as a porous membrane. The membrane forms a substrate onto which an extracellular matrix (ECM) and cell layers may be defined. ECM is as a protein environment that may act as a base layer for the cells to grow on. The membrane may thus form an interface for the cellular cross talk of endothelial and epithelial cell layers defined on either side of the membrane.

The microfluidic device comprises a top plate, a bottom plate and a membrane. The top plate comprises a top channel arranged on a first surface of the top plate.

The bottom plate comprises a bottom channel arranged on a first surface of the bottom plate. The top channel is overlapping the bottom channel over an entire length of the top channel.

The first surface of the top plate and the first surface of the bottom plate are facing each other, such that the top channel and the bottom channel are facing each other. The membrane is arranged between the top plate and the bottom plate so that the top channel and the bottom channel are separated by the membrane. The top channel and the bottom channel are configured to receive a respective fluid flow. When the top channel and the bottom channel receives the respective fluid flow, the respective fluid flows of the top channel and the bottom channel are separated by the membrane. In other words, a fluid flow through the top channel and a fluid flow through the bottom channel flow on opposite sides of the membrane. The membrane may thus act as a barrier between the top channel and the bottom channel.

The top plate comprises a top channel inlet port being in fluid communication with the top channel.

The top plate comprises a first bottom channel inlet port and a second bottom channel inlet port. The first bottom channel inlet port and the second bottom channel inlet port are both in fluid communication with the bottom channel. The second bottom channel inlet port is arranged further from the longitudinal center of the bottom channel, such as closer to an edge of the bottom plate, such as further away from the center of the bottom plate, such as further from the top channel inlet port, than the first bottom channel inlet port.

The first bottom channel inlet port may, in one or more example microfluidic devices, be configured as a dedicated inlet port for cell seeding in the bottom channel. The first bottom channel inlet port may herein also be referred to as a cell seeding port. The second bottom channel inlet port may, in one or more example microfluidic devices, be configured as a flow inlet port for a fluid flow through the microfluidic device. The fluid flow may be a culture media flow, which is flowed over both sides of the membrane, thereby ensuring that the cells seeded onto both sides of the membrane are supplied with culture medium and that metabolic waste is removed from the cells. As the cells adhere to the membrane, proliferate and form a confluent layer, such as a confluent cell layer, on both sides of the membrane, an OOC system is formed that, in combination with a media flow, such as a fluid flow, through the top and bottom channels, can mimic the organ tissue response to external stimuli. To mimic the cell function closely, relevant shear and tensile stresses can be applied to the cells, thereby simulating the shear and tensile stresses that would be exerted on the cells in a living system, such as in the human gut system. The fluid flow through the channels provides the shear stress on the cells, which results in more accurate cell function replication.[3-5] By providing the first bottom channel inlet port in the vicinity of the top channel inlet port, the tissue barrier created by the cells is mostly formed between the top channel and the bottom channel instead of being formed in an unnecessarily long bottom channel if the cells were to be seeded via the second bottom channel inlet port, which has a smaller percentage of overlap with the top channel.

In one or more example microfluidic devices, the top plate comprises a top channel outlet port being in fluid communication with the top channel.

In one or more example microfluidic devices, the top plate comprises a first bottom channel outlet port being in fluid communication with the bottom channel.

In one or more example microfluidic devices, the top plate comprises a second bottom channel outlet port being in fluid communication with the bottom channel. The second bottom channel outlet port may be arranged further from the longitudinal center of the bottom channel, such as closer to an edge of the bottom plate, such as further away from the center of the bottom plate, such as further from the top channel inlet port, than the first bottom channel outlet port.

In one or more example microfluidic devices, the top channel inlet port, the first bottom channel inlet port, the second bottom channel inlet port, the top channel outlet port, the first bottom channel outlet port and/or the second bottom channel outlet port are arranged perpendicular to the second surface of the top plate. The ports being arranged perpendicular to the second surface of the top plate herein means that a center axis of the ports extends perpendicular to the second surface of the top plate. In one or more example microfluidic devices, one or more of the inlet ports and/or outlet ports may be arranged at an angle to the second surface of the top plate. For example, the second bottom inlet port and/or the second bottom outlet port may be arranged at an angle to the longitudinal axis of the bottom channel, such as at an angle in the range of 45-90°. By arranging the second bottom inlet port and/or the second bottom outlet port at an angle to the longitudinal axis, the fluid flow through the channel may be improved since sharp direction changes in the flow path can be reduced, which may otherwise lead to a nonlaminar flow at these points.

The inlet and outlet ports used for cell seeding, such as the top channel inlet and top channel outet ports and the first bottom channel inlet and first bottom channel outlet ports may, in one or more example microfluidic devices, be arranged perpendicular to the longitudinal axis of the top channel and/or bottom channel in order to reduce the length of the inlet ports. By reducing the length of the inlet ports, the length of the sections of the microfluidic device where cells may adhere without being able to form a confluent cell layer on the membrane may be reduced. Thereby, the cells may be focused towards the membrane, so that a higher percentage of the seeded cells can be used for replicating the cell function of an organ in the microfluidic device.

In one or more example microfluidic devices, the top plate comprises a bottom channel sensor port, such as one or more bottom channel sensor ports, for receiving a sensor for monitoring the bottom channel. The bottom channel sensor port is in fluid communication with the bottom channel.

In one or more example microfluidic devices, the top plate comprises a top channel sensor port, such as one or more bottom channel sensor ports, for receiving a sensor for monitoring the top channel. The bottom channel sensor port is in fluid communication with the bottom channel.

The ports, such as the top channel inlet port, the first bottom channel inlet port, the second bottom channel inlet port, the top channel outlet port, the first bottom channel outlet port, the second bottom channel outlet port, the bottom channel sensor port and/or the top channel sensor port, may be luer ports, such as mini luer ports. The luer ports may be female luer fittings and/or male luer fittings. In one or more example microfluidic devices, the distance between a center axis of a first port and a second port may be a multiple of 4.5 mm. Other types of ports can be used as well, and the particular type of port is not limiting.

In one or more example microfluidic devices, the top channel sensor port and/or the bottom channel port comprises a raised portion protruding from a second surface of the top plate, wherein the top sensor port protrudes further from the second surface than the bottom sensor port. The height of the raised portion may correspond to the difference in distance from the top plate between the top channel and the bottom channel. Thereby, the same type of sensor can be fitted to both the top channel and the bottom channel without protruding beyond the top channel which is located closer to the top plate than the bottom channel.

Gut cells form three-dimensional (3D) structures (also referred to as villi), which can be up to 1.5 mm high in a human, however, in OOC devices the gut cells are typically shorter. Therefore, the upper range of the height of the channel may be defined by the villi height plus space for a fluid flow on top of these villi structures.

In one or more example microfluidic devices, the top channel has a height in the range of 0.2 to 2 mm. In one or more example microfluidic devices, the bottom channel has a height in the range of 0,05 to 1.5 mm, such as in the range of 0.1 to 0.5 mm. In one or more example microfluidic devices, the top channel and/or the bottom channel has a width in the range of 0.25 to 2 mm.

In one or more example microfluidic devices, the top channel and/or the bottom channel has a length being a multiple of 4.5 mm. In one or more example microfluidic devices, the first bottom channel inlet port, the second bottom channel inlet port and/or the top channel inlet port are arranged at a distance being a multiple of 4.5 mm from each other. The length may be a multiple of 4.5 mm to keep the inlet spacing at a pitch of standard pipetting robots. Thereby, the microfluidic device can be compatible to standard pipetting robots configured for receiving for example 96 well plates, which are built around pitches being multiples of 4.5 mm.

In one or more example microfluidic devices, the channels are not arranged in straight line, but are curved, such as are arranged as a serpentine. In this case the length of the channel may not be a multiple of 4.5 mm, instead the ports are arranged at a distance from each other being a multiple of 4.5 mm.

In one or more example microfluidic devices, one or more of the top part, the bottom part and the membrane may be made out of polydimethylsiloxane (PDMS). In one or more example microfluidic devices, the top part, the bottom part and the membrane are all made out of PDMS. In one or more example microfluidic devices, the membrane is made out of PDMS. PDMS has the benefit that it has a low cost, high transparency in the visible region and has rapid prototyping capabilities.[7] Having rapid prototyping capabilities herein means that it allows for fast fabrication of one or more physical parts, model or assembly, for example using 3D computer aided design (CAD). The creation of the part, model or assembly may be assisted using additive manufacturing, more commonly known as 3D printing.

However, PDMS may absorb small, lipophilic molecules (such as pirfenidone, rhodamine B and others) and/or extracellular vesicle-specific intercalating dye. PDMS may absorb as much as 0.4% of its' mass with a channel cross-sectional area of $7.1 mm^2$ of a drug-like molecule leading to a severe concentration drop in the channel from $2 \mu M$ to $20 nM$.[8] When utilizing PDMS as material in the microfluidic device, the absorption and release by the material may be simulated to compensate for the absorbtion when performing experiments using the microfluidic device. [4,9]

In one or more example microfluidic devices, the top plate and/or the bottom plate is made out of a thermoplastic material, such as one or more of cyclic olefin copolymer (COC), polystyrene (PS), and polycarbonate (PC). These materials can have a lower gas permeability, such as an oxygen and/or water permeability, than PDMS. For example, while PDMS has an oxygen barrier of around $3 \times 10^4 \frac{cm^3 \cdot mm}{m^2 \cdot d \cdot atm}$, this value for COC is $17 \frac{cm^3 \cdot mm}{m^2 \cdot d \cdot atm}$, for PS is $102 \frac{cm^3 \cdot mm}{m^2 \cdot d \cdot atm}$, and for PC is $90 \frac{cm^3 \cdot mm}{m^2 \cdot d \cdot atm}$. By making the microfluidic device out of one or more of COC, PS, and/or PC, the small molecule absorption and/or gas permeability of the top plate and/or the bottom plate can be reduced. This is benficial since a significant profile of modern drugs are small, lipophilic molecules, which, upon introduction in highly absorbent channels, would be absorbed in channel side walls, subsequently reducing the actual molecule concentration in the channels. Furthermore, since the small molecule absorption and/or gas permeability is reduced, no compensation for absorption and/or gas permeability may be required. Thereby, a more accurate picture of the performed experiment may be provided, which subsequently may improve the result of the performed experiments. Using thermoplastic materials also allows the inlet ports and/or outlet ports to be integrally formed in the top plate.

In one or more example microfluidic devices, other materials can be used as well, such as other polymers, and the disclosure is not so limited.

In one or more example microfluidic devices, the top plate and/or the bottom plate are injection molded. A benefit of injection molding the top plate and/or bottom plate is that the cycle times for manufacturing of each part can be reduced. Thereby the production rate of the microfluidic device may be increased.

Furthermore, by using injection molding for manufacturing the top plate and/or the bottom plate, manufacturing facility requirements for clean rooms can be less stringent as the device manufacturing process is limited to a single tool, such as to an injection molding machine, such as an injection press. In one or more example microfluidic devices, other methods of manufacturing can be used as well.

By using injection molding for manufacturing the parts of the microfluidic device, such as the top plate and/or the bottom plate, thin (such as in the range of 0.6-1 mm) yet rigid (such as having a Young's modulus of at least 1 MPa) parts suitable for simulating organ functions can be produced. Such thin parts are suitable for use with high magnification objectives. Since thermoplastic materials are less gas permeable than for example PDMS, the parts can be made thinner, which improves microscopy due to lower transmission losses within the material and thereby enables a use of low, such as small, working distance objectives. High magnification objectives used for microscopy typically have low working distances, such as the physical distance between the most outward point of the lens to a first depth of a field plane. Typically, this distance may be between 0.1 - 1 mm. It is therefore beneficial to reduce the thickness of the parts to ensure that the first depth of the field plane is located at the membrane.

In one or more example microfluidic devices, the thickness of the bottom plate and/or the top plate may be as thin as can be reliably manufactured, such as in the range of 0.6-1 mm, such as 0.8 mm. The height of the bottom channel may be in the range from 0.1 to 0.5 mm, where a higher channel height results in an increase in the bottom plate thickness. The width and the length of the bottom channel may correspond to the top channel. By reducing the thickness of the bottom plate and/or the top plate higher magnification objectives may be used for studying the cells seeded in the channels. OOC-devices made out of PDMS typically require a bottom thickness of at least 1 mm for structural rigidity, which means that a higher magnification (such as a magnification of 40x or higher, sometimes even a magnification of 20x) objectives cannot be used, which limits the cell studies that can be performed on a PDMS OOC-device.

In one or more example microfluidic devices, the membrane is made out of a thermoplastic, such as one or more of cyclic olefin copolymer (COC), polyethylene terephthalate (PET), and polycarbonate (PC). The membrane may be an etched membrane, such as a track-etched membrane. Etching the membrane allows a pore dimension of the surface of the membrane to be controlled during manufacturing.

By using thermoplastics, such as COC, PS, PET and/or PC, for the top plate, the bottom plate, and/or the membrane, the microfluidic device may be bonded together using ultrasonic welding.

In one or more example microfluidic devices, the top plate and/or the bottom plate and/or the membrane are bonded together using ultrasonic welding. Ultrasonic welding may be used to bond the top plate, the bottom plate and/or the membrane together to a solid microfluidic device. By using ultrasonic welding to bond the parts of the microfluidic device together, a cycle time for bonding the parts can be reduced compared to other bonding solutions, such as PDMS based fabrication processes. A typical fabrication process for attaching PDMS to a thermoplastic membrane may be done via epoxy-amine bond. Epoxy-amine bond may be achieved via surface modification of the PDMS parts and thermoplastic parts with epoxy and amine functionalities, respectively. This involves a higher number of fabrication steps than the ultrasonic welding methods and therefore a longer cycle time for the fabrication process.

By using ultrasonic welding to bond the parts together, the cycle time for bonding the top plate, the bottom plate and/or the membrane together may be reduced to less than 10s, in comparison to a cycle time of several minutes to multiple hours for previous methods. Another advantage of ultrasonic welding is that an alignment of the parts to be bonded can be simplified, as no surface functional molecules must be bonded to the membrane in contrast to other manufacturing processes, such as the PDMS fabrication process. By using ultrasonic welding to bond the top plate and the bottom plate with the membrane, a monolithic block can be formed, thereby reducing, such as preventing a gas ingress, in the microfluidic device between the top plate and the bottom plate compared to microfluidic devices using other manufacturing processes where the microfluidic device after assembly still comprises of a plurality of distinct parts.

Current bonding techniques have the problem that the membrane may crease and/or buckle during assembly and bonding of the OOC devices. Buckling and/or creasing of the membrane may cause issues with cell growth on the membrane and microscopy of the cells and/or membrane. To ensure that the membrane is held in place and/or is stretched during the high frequency oscillations caused by the ultrasonic welding, the top plate and/or the bottom plate may, in one or more example microfluidic devices, comprise tensioning elements for tensioning the membrane. By stretching the membrane mechanical stress in the form of peristaltic membrane stretching may be exerted on cells seeded onto the membrane, thereby providing a more accurate cell function replication.

In one or more example microfluidic devices, the top plate and/or the bottom plate comprises a first tensioning element for tensioning the membrane during assembly of the microfluidic device.

In one or more example microfluidic devices, the top plate and/or the bottom plate comprises a second tensioning element, the first tensioning element and the second tensioning element being arranged on opposite sides of the top channel and/or the bottom channel, such that the membrane is stretched between the tensioning elements such that the membrane is stretched over the top channels and the bottom channels during assembly of the microfluidic device.

In one or more example microfluidic devices, the tensioning elements may comprise one or more protrusions formed on a membrane facing surface of the top plate and/or the bottom plate, such as the first surface of the top plate and/or the first surface of the bottom plate. In one or more example microfluidic devices, the tensioning elements may comprise one or more indentations for receiving the protrusions, the indentations being arranged on the membrane facing surface of the bottom plate and/or the top plate opposite of the surface having protrusions.

In other words, when the first surface of the top plate comprises protrusions, the first surface of the bottom plate may comprise indentations for receiving the protrusion arranged on the first surface of the top plate. Correspondingly, when the first surface of the bottom plate comprises protrusions, the first surface of the top plate may comprise indentations for receiving the protrusion arranged on the first surface of the bottom plate. The protrusions and/or the indentations may extend along the length of the channels. When the top plate is pressed against the bottom plate during the assembly process, the protrusions press the membrane into the indentations. By arranging the tensioning elements on opposite sides of the channels, such as a protrusion and a corresponding indentation on each side of the channels, pressing the membrane into each of the indentations by means of the respective protrusions causes the membrane to stretch over the channel. In one or more example microfluidic devices, the tensioning elements may be configured to tension the membrane so that a deflection of the membrane is limited to a maximum of $20\mu m$. In one or more example microfluidic devices, the tensioning elements may be configured to tension the membrane so that a deflection of the membrane is limited to a maximum of $15\mu m$. In one or more example microfluidic devices, the tensioning elements may be configured to tension the membrane so that a deflection of the membrane is limited to a maximum of $10\mu m$. In one or more example microfluidic devices, the protrusions may act as energy directors that melt away during the ultrasonic welding process and welds the membrane to the top plate and/or the bottom plate.

In one or more example microfluidic devices, the top plate comprises a plurality of top channels and the bottom plate comprises a plurality of bottom channels.

In one or more example microfluidic devices, the top channels and the bottom channels extend in a longitudinal direction of the top plate and the bottom plate respectively. The longitudinal direction of the top plate and/or the bottom plate is a direction parallel to the longest outer edge of the top plate and/or the bottom plate.

In one or more example microfluidic devices, the plurality of top channels and/or bottom channels have the same height, length and/or width. In one or more example microfluidic devices, the plurality of top channels and/or the plurality of bottom channels have the same dimensions. Thereby a single microfluidic device can comprise multiple substantially identical OOC devices (where each respective combination of top channel and bottom channel constitutes a single OOC device simulating a single organ). By having a plurality of substantially identical OOC devices on a single microfluidic device more replications of a simulations on an organ can be performed on each microfluidic device, which can increase the output of the simulation.

In one or more example microfluidic devices, the plurality of top channels and/or the plurality of bottom channels have different dimensions (such as different heights, lengths, and/or widths), to simulate different parts of an organ, such as of the gut, which may not all be of the same length, height or width. By varying the height, length, and/or the width of the plurality of top channels and/or bottom channels, the differences in the organ parts volume and/or surface area, such as of the various parts of the gut, can be represented in the microfluidic device. In one or more example microfluidic devices, each of the plurality of top channels and/or bottom channels is configured to simulate a respective part of the gut, such as the duodenum, the jejunum, the appendix, the cecum, the colon, and the rectum. Since each part of the organ has, effectively, a different surface area, the most accurate representation of the organ in the microfluidic device, such as in an OOC device, is a microfluidic device having interconnected channels with different dimensions.

The location of the inlet ports and the outlet ports for each of the plurality of top channels and/or bottom channels may be determined based on a flow direction through each of the channels. For example, the flow direction through a first top channel and a first bottom channel may be opposite to a flow direction through a second top channel and a second bottom channel being arranged adjacent to the first top channel and first bottom channel. A top channel and an overlapping bottom channel may herein be referred to as a pair of channels or a channel pair. In other words, in one or more example microfluidic devices, the microfluidic device may comprise a plurality of pair of channels. Each pair of channels may comprise a plurality of bottom channel ports and top channel ports being symmetrically arranged along the top and/or bottom channels. For example, each channel pair may comprise a pair of top channel ports arranged at opposite ends of the top channel, a pair of first bottom channel ports and a pair of second bottom channel ports, wherein the pair of second bottom channel ports are arranged at opposite ends of the bottom channel and the pair of first bottom channel ports are arranged between a respective top channel port and a respective second bottom channel port. Which of the top channel ports, the first bottom channel ports and the second bottom channel ports are inlets and which are outlets can be determined based on the flow direction through the channels, where the inlet ports are arranged upstream of the outlet ports of each channel and the outlet ports are arranged downstream of the inlet ports.

In one or more example microfluidic devices, the microfluidic device comprises a connector for interconnecting the plurality of top channels and/or the plurality of bottom channels. The connector may be configured to connect a first top channel with a second top channel and/or to connect a first bottom channel with a second bottom channel. In one or more example microfluidic devices, the connector may be configured to connect a first top channel with a second bottom channel, such as a top channel of a first pair of channels with a bottom channel of a second pair of channels. By interconnecting the plurality of top channels and/or bottom channels more complex organs having different parts, such as the gut, can be simulated. The connector may be configured to connect, such as fluidly connect, an outlet port from a first top channel and/or a first bottom channel to an inlet port of a second top channel and/or a second bottom channel. By connecting the plurality of top channels and/or bottom channels using a plurality of connectors a continuous fluid flow can be generated through the plurality of top channels and/or bottom channels. In one or more example microfluidic devise, the connector is made from thermoplastic, such as one or more of COC, PS, PET and/or PC, which may allow physiological gas concentrations to be retained through the connector.

In one or more example connectors, the connector may comprise two parts, such as a top connector part and a bottom connector part. The two parts of the connector may, in one or more example connectors, be bonded together to form one integral connector. In one or more example connectors, the two parts of the connector may be formed as a single integral piece.

The bottom connector part may comprise a first fitting and a second fitting, such as a first and a second luer fitting, for interfacing with the inlets and/or outlets of the microfluidic device. The first fitting and the second fitting may be a male and/or a female luer fitting. The first fitting and the second fitting may be hollow, so that a microfluidic channel is provided in the first and the second fitting.

The bottom connector part may comprise a microfluidic channel, connecting the first fitting and the second fitting, through which microfluidic channel a fluid may flow between the first fitting and the second fitting. In one or more example connectors, the top connector part is transparent, to allow visual inspection of the flow through the connector. In one or more example connectors, the top connector part can comprise a microfluidic channel through which microfluidic channel a fluid may flow between the first fitting and the second fitting.

In one or more example connectors, the top connector part is a cover covering the microfluidic channel comprised in the connector.

In one or more example connectors, the connector may comprise an integrated sensor. In one or more example connectors, the connector comprises a port, such as a sensor port, for receiving a sensor for measuring a flow, such as a fluid flow, through the connector. The port for receiving the sensor may be a luer port, such as a mini-luer port. The sensor for measuring the flow through the connector, may be a sensor measuring characteristics of the flow through the connector. In one or more example microfluidic devices the sensor may be a sensor for gas and/or analyte sensing. The sensor for measuring the flow through the connector may be a luer-based, such as a mini-luer based, sensor. The sensor may be one or more of an oxygen sensor, and a Transepithelial Electrical Resistance (TEER) sensor. The oxygen sensor may in one or more example microfluidic devices be used for monitoring, such as real-time monitoring, of cell metabolism of the cell culture in the channels, and/or for a feedback loop, such as a real-time feedback loop, of culturing media composition in the channels. The TEER sensor may in one or more example microfluidic devices be used for monitoring, such as real-time monitoring, of a cell barrier integrity in the channels, for a feedback loop of barrier formation in the channels, and/or for impedance spectroscopy read-out from the channels.

In one or more example microfluidic devices, the distance between a center axis of the first fitting and/or the second fitting of the connector is a multiple of 4.5 mm.

In one or more example microfluidic devices, the plurality of top channels and the plurality of bottom channels are arranged in parallel to each other. The plurality of top channels and the plurality of bottom channels may be arranged at a predetermined distance from each other. The predetermined distance may correspond to a distance between the first fitting and the second fitting of the connector.

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the disclosure or as a limitation on the scope of the disclosure. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

The figures are schematic and simplified for clarity, and they merely show details which aid understanding the disclosure, while other details have been left out. Throughout, the same reference numerals are used for identical or corresponding parts.

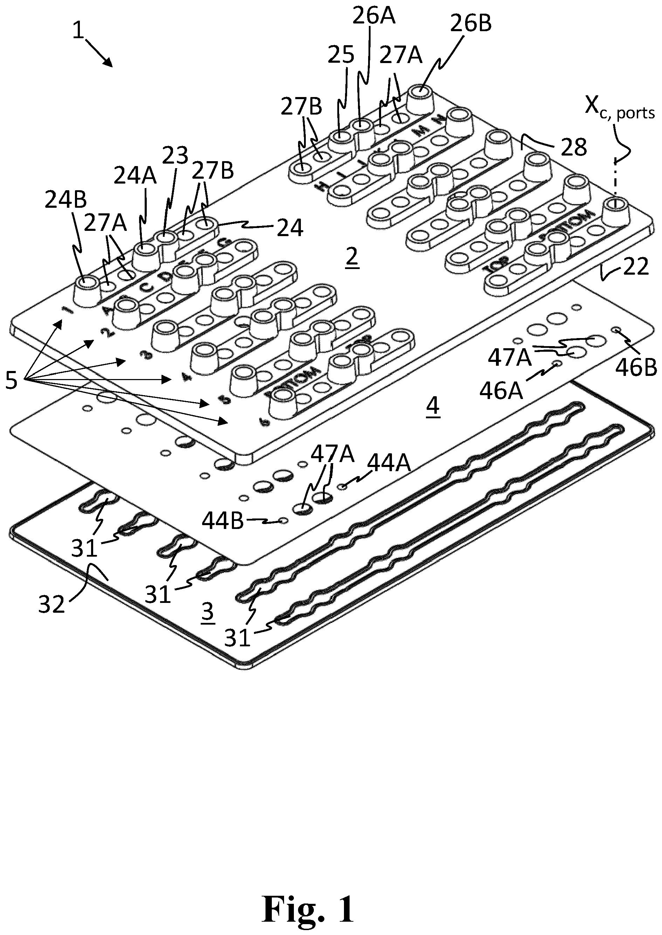

Fig. 1 illustrates an exploded view of an example microfluidic device 1 for simulating organ functions according to the current disclosure. The microfluidic device 1 comprises a top plate 2, a bottom plate 3 and a membrane 4. The top plate 2 comprises a top channel (not shown in Fig. 1) arranged on a first surface 22 of the top plate 2.

The bottom plate 3 comprises a bottom channel 31 arranged on a first surface 32 of the bottom plate 3. The top channel 21 is overlapping the bottom channel 31 over an entire length of the top channel 21.

The example microfluidic device 1 shown in Fig. 1 comprises six pair of channels 5, such as six pair 5 of overlapping top channels (not shown in Fig. 1) and bottom channels 31 separated by the membrane 4, on the microfluidic device 1. The six pair of channels 5 may, in one or more example microfluidic devices, be used individually, so that each pair of channels represents a separate organ. The six pair of channels 5 may, in one or more example microfluidic devices, be interconnected, for example by means of a connector, to model various regions of the organ, such as various regions of the gut, on the same microfluidic device, rather than having separate microfluidic device for each of the region of the organ. This may be done without compromising easy loading and cell seeding on the chip allowed by single channel systems.

The first surface 22 of the top plate 2 and the first surface 32 of the bottom plate are facing each other, such that the top channel (not shown in Fig. 1) and the bottom channel 31 are facing each other. The membrane 4 is arranged between the top plate 2 and the bottom plate 3 so that the top channel and the bottom channel 31 are separated by the membrane 4. The top channel and the bottom channel 31 are configured to receive a respective fluid flow, such as a liquid flow and/or gas flow. When the top channel and the bottom channel 31 receives the respective fluid flow, the respective fluid flows of the top channel and the bottom channel 31 are separated by the membrane 4. In other words, a fluid flow through the top channel and a fluid flow through the bottom channel 31 flow on opposite sides of the membrane 4.

The top plate 2 comprises a top channel inlet port 23 being in fluid communication with the top channel.

The top plate 2 comprises a first bottom channel inlet port 24A and a second bottom channel inlet port 24B. The first bottom channel inlet port 24A and the second bottom channel inlet port 24B are both configured to be in fluid communication with the bottom channel 31. The second bottom channel inlet port 24B is arranged further from the longitudinal center of the bottom channel 31 than the first bottom channel inlet port 24A. In the example microfluidic device shown in Fig. 1, the first bottom channel inlet port 24A is arranged between the second bottom channel inlet port 24B and the top channel inlet port 23.

The first bottom channel inlet port 24A may, in one or more example microfluidic devices, be configured as a dedicated inlet port for cell seeding in the bottom channel, such as a cell seeding port. The second bottom channel inlet port 24B may, in one or more example microfluidic devices 1, be configured as a flow inlet port for a fluid flow through the bottom channel of the microfluidic device 1, which fluid flow exerts a shear stress on cells seeded in the bottom channel and supplies the cells with fresh medium, provides an environment for circulating cells or molecular and cellular signals, while removing metabolic waste. By providing the first bottom channel inlet port in the vicinity of the top channel inlet port the tissue barrier created by the cells is mostly formed between the top channel 21 and the bottom channel 31, instead of being formed in an unnecessarily long bottom channel 31 if the cells were to be seeded via the second bottom channel inlet port 24B, which has a smaller percentage of overlap with the top channel 21.

In the example microfluidic device 1 shown in Fig. 1, the top plate 2 comprises a top channel outlet port 25 being in fluid communication with the top channel 21. The top plate 2 comprises a first bottom channel outlet port 26A and a second bottom outlet port 26B being in fluid communication with the bottom channel 31. The location of the inlet ports and the outlet ports may be determined based on the flow direction through each of pair 5 of channels. In the example microfluidic device 1 shown in Fig. 1, the flow direction through the upmost pair 5 of channels is from left to right in the figure, such that the flow is from the inlet ports 23, 24A, 24B to the outlet ports 25, 26A, 26B. However, the flow may in one or more example microfluidic devices 1 be in the opposite direction such that the ports, 25, 26A, 26B act as inlet ports, and the ports 23, 24A, 24B act as outlet ports. The flow direction through the pair 5 of channels may, in one or more example microfluidic devices alternate, such that the flow direction through every second pair of channels is opposite to the flow direction through the previous pair of channels. The flow direction through each pair of channels, such as through the bottom channel and the top channel of each pair of channels may be the same.

In the example microfluidic device 1 of Fig. 1, the top plate 2 comprises a second bottom channel outlet port 26B being in fluid communication with the bottom channel 31. The second bottom channel outlet port 26B is arranged further from the longitudinal center of the bottom channel 31 than the first bottom channel outlet port 26A.

In the example microfluidic device 1 of Fig. 1, the top channel inlet port 23, the first bottom channel inlet port 24A, the second bottom channel inlet port 24B, the top channel outlet port 25, the first bottom channel outlet port 26A and/or the second bottom channel outlet port 26B are arranged perpendicular to the second surface 28 of the top plate 2. The ports being arranged perpendicular to the second surface 28 of the top plate 2 herein means that a center axis $X_{c,ports}$ of the ports extends perpendicular to the second surface 28 of the top plate 2.

In the example microfluidic device 1 of Fig. 1, the top plate 2 comprises a bottom channel sensor port 27A, such as one or more bottom channel sensor ports 27A, for receiving a sensor for monitoring the bottom channel 31.

In the example microfluidic device 1 of Fig. 1, the top plate 2 comprises a top channel sensor port 27B, such as one or more bottom channel sensor ports 27B, for receiving a sensor for monitoring the top channel 21.

The ports, such as the top channel inlet port 23, the first bottom channel inlet port 24A, the second bottom channel inlet port 24B, the top channel outlet port 25, the first bottom channel outlet port 26A, the second bottom channel outlet port 26B, the bottom channel sensor port 27A and/or the top channel sensor port 27B, may be luer ports, such as mini luer ports.

In the example microfluidic device 1 of Fig. 1, the membrane 4 comprises a first bottom inlet opening 44A, a second bottom inlet opening 44B, a first bottom outlet opening 46A, a second bottom outlet opening 46B for allowing a fluid to flow through the membrane 4 between the bottom channel and the first bottom channel inlet port 24A, the second bottom channel inlet port 24B, the first bottom channel outlet port 26A and the second bottom channel outlet port 26B through the membrane 4 into the bottom channel 31 of the bottom plate 3. The membrane 4 also comprises a plurality of sensor openings 47A for allowing a sensor arranged in the bottom sensor ports 27A to protrude through the membrane 4 into the bottom channel 31.

In the example microfluidic device 1 of Fig. 1, the top channel sensor port 27B comprises a raised portion 29 protruding from a second surface 28 of the top plate 2, wherein the top sensor port 27B protrudes further from the second surface than the bottom sensor port 27A.

In the example microfluidic device 1 of Fig. 1, the top channels 21 and the bottom channels 31 extend in a longitudinal direction of the top plate 2 and the bottom plate 3 respectively.

In the example microfluidic device 1 of Fig. 1, the plurality of top channels 21 have the same height, length and/or width. In the example microfluidic device 1 of Fig. 1, the plurality of bottom channels 31 may also have the same height, length and/or width.

The top plate 2 and/or the bottom plate 3 may be made out of one or more of COC, PS, and PC. The membrane 4 may be made out of one or more of COC, PET, and PC.

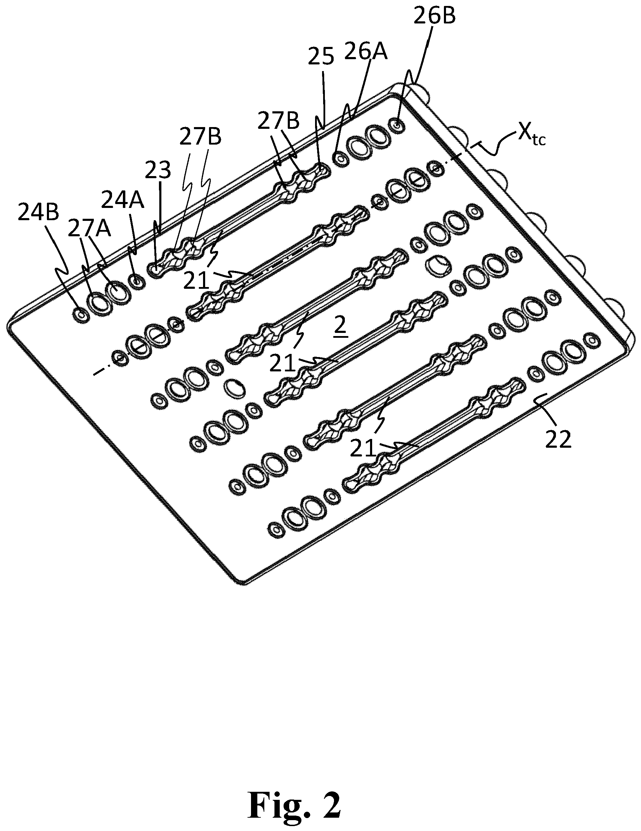

Fig. 2 is a perspective view of the membrane facing first surface 22 of the top plate 2 of the microfluidic device 1 shown in Fig. 1. As can be seen in Fig. 2, the top plate comprises a plurality of top channels 21 arranged on the first surface 22 of the top plate. In the example top plate shown in Fig. 2 the top plate comprises six top channels 21 arranged in parallel to each other. Each of the top channels 21 extend between a respective top channel inlet port 23 and a respective top channel outlet port 25. The bottom channel inlet ports 24A, 24B and the bottom channel outlet ports 26A, 26B associated with each bottom channel are arranged along an extension of a longitudinal axis $X_{tc}$ of a respective top channel 21.

The first bottom channel inlet port 24A, the second bottom channel inlet port 24B, the top channel inlet port 23, the top channel outlet port 25, the first bottom channel outlet port 26A, and/or the second bottom channel may be arranged at a distance being a multiple of 4.5 mm from each other.

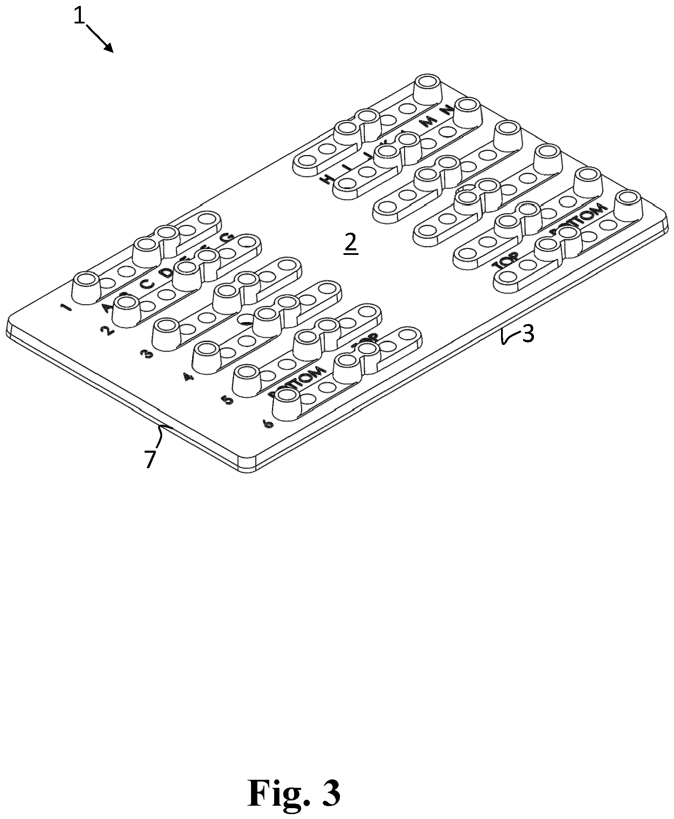

Fig. 3 illustrates a perspective view of an assembled example microfluidic device 1 according to the current disclosure. The top plate 2, the bottom plate 3 and the membrane (not shown in Fig. 3) may be bonded together to form a solid structure, for example using ultrasonic welding. By using ultrasonic welding to bond the top plate and the bottom plate with the membrane, a monolithic block can be formed, which prevents gas ingress into the microfluidic device between the top plate 2, the bottom plate 3 and/or the membrane 4. The top plate 2 and the bottom plate 3 may be bonded together by means of a weld 7 going around the entire perimeter of the microfluidic device 1, wherein the weld may be formed during ultrasonic welding. Providing a weld 7 going around the entire perimeter of the microfluidic device 1 ensures that even if there would be a leak in any of the channels of the microfluidic device 1, such as in any of the top channels 21 and/or bottom channels 31, the fluid cannot escape the microfluidic device 1.

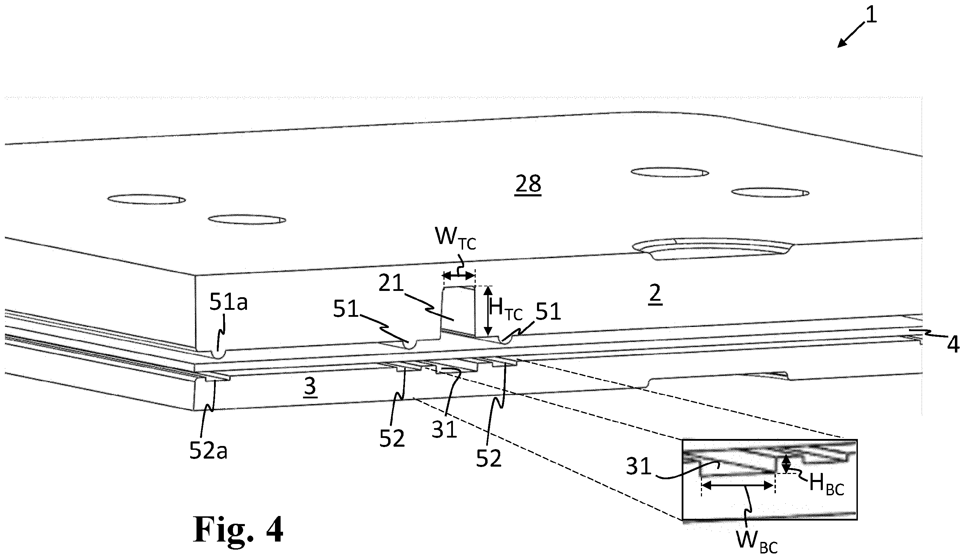

Fig. 4 is a perspective view of a cross-section of an example microfluidic device 1 according to the current disclosure, seen in a longitudinal direction of the top channel 21 and the bottom channel 31. The top channel 21 and the bottom channel 31 are arranged so that they overlap over the entire length of the top channel 21 and are separated by the membrane 4.

To ensure that the membrane 4 is held in place and/or is stretched during assembly, such as during ultrasonic welding, the top plate 2 and the bottom plate 3 comprise tensioning elements 51, 52 for tensioning the membrane 4.

In the example microfluidic device 1 shown in Fig. 4, the top plate 2 comprises a plurality of first tensioning elements 51 and the bottom plate 3 comprises a plurality of second tensioning elements 52. In one or more example microfluidic devices, the first tensioning element 51 may be a protrusion, such as a ridge, formed on the membrane facing surface 22 of the top plate 2. When the first tensioning element 51 is a protrusion, the first tensioning element 51_may, in one or more example microfluidic devices, be wedge shaped, such that the first tensioning element 51, such as a cross section of the first tensioning element 51, is wider at a proximal end of the first tensioning element 51 than at a distal end, wheren the proximal end is the end closest to the membrane facing surface 22 and the distal end is the end furthest away from the membrane facing surface 22. In other words, the width of the first tensioning element 51 may decrease from the membrane facing surface towards the bonding surface in the second tensioning element 52. The second tensioning element 52 may be an indentation arranged on the membrane facing first surface 31 of the bottom plate 3 for receiving the protrusion of the first tensioning element 51. In one or more example methods, tensioning elements may be arranged on opposite sides of the top channel and/or the bottom channel. Thereby, the the membrane may be stretched between the tensioning elements arranged on opposite sides of the top channel and/or the bottom channel, such that the membrane is stretched over the top channels and the bottom channels during assembly of the microfluidic device.

The protrusions 51 and/or the indentations 52 may extend along the length of the top channel 21 and the bottom channel 31 respectively. When the top plate 2 is pressed against the bottom plate 3 during the assembly process, the protrusions 51 press the membrane 4 into the indentations 52. In the example microfluidic device 1 shown, in Fig. 4, tensioning elements are arranged on opposite sides of the top channel 21 and the bottom channel. In the example microfluidic device of Fig. 4 a protrusion 51 is arranged on each side of the top channel and a corresponding indentation 52 is arranged on each side of the bottom channel, such that the membrane 4 is pressed into each of the indentations 52 by means of the respective protrusions 51, so that the membrane is stretched over the top channel 21 and the bottom channel 31. The protrusions may further act as energy directors that melt away during the ultrasonic welding process and welds the membrane 4 to the top plate 2 and/or the bottom plate 3. A further protrusion 51a and corresponding indentation 52a may be arranged along the outer edge of the top plate 2 and the bottom plate 3 respectively.

The top channel 21 may have a height $H_{TC}$ in the range of 0.2 to 2 mm. The bottom channel 31 may have a height $H_{BC}$ in the range of 0.1 to 0.5 mm. The top channel 21 may have a width $W_{TC}$ in the range of 0.15 to 2.5 mm, such as in the range of 0.25 to 2 mm. The bottom channel 31 may have a width $W_{BC}$ equal to the width $W_{TC}$ of the top channel, such as in the range of 0.15 to 2.5 mm, such as in the range of 0.25 to 2 mm.

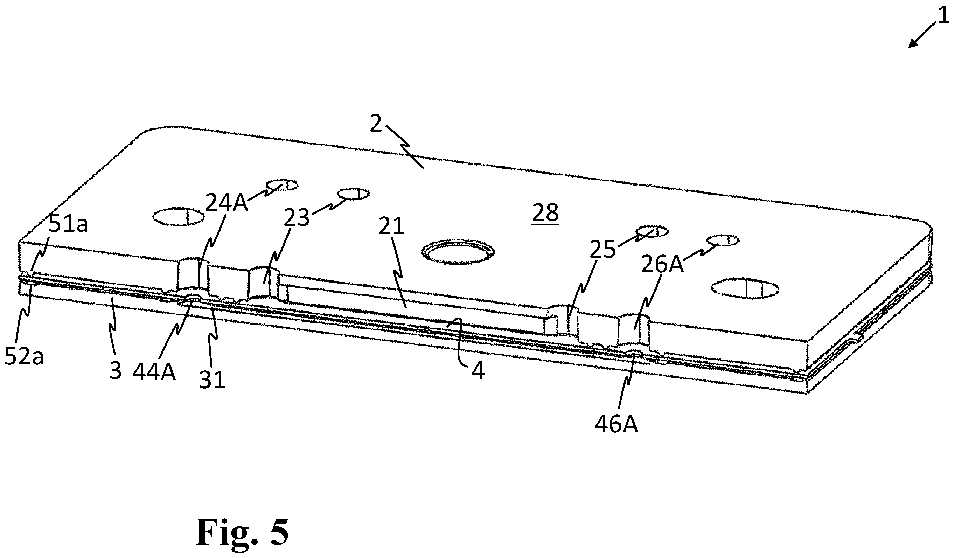

Fig. 5 is a perspective view of a cross-section of an assembled example microfluidic device 1 according to the current disclosure seen in a direction perpendicular to the longitudinal direction of the top channel 21 and the bottom channel 31. In Fig. 5 the arrangement of an example top inlet port 23, an example top outlet port 25, an example bottom inlet port 24A and an example bottom outlet port 26A on the top plate 1 is shown. The top inlet port 23, the top outlet port 25, the bottom inlet port 24A and the bottom outlet port 26A are placed directly above the respective channel 21, 31, such that the top inlet port 23, the top outlet port 25, the bottom inlet port 24A and the bottom outlet port 26A are arranged perpendicular to longitudinal extension of the top channel 21 and/or bottom channel 31. By placing the top inlet port 23, the top outlet port 25, the bottom inlet port 24A and the bottom outlet port 26A directly above the top channel 21 and/or bottom channel 31 instead of placing the inlets and/or outlets to either side of the top channel 21 and/or bottom channel 31, the top inlet port 23 and the bottom inlet port 24A, as well as the top outlet port 25 and the bottom outlet port 26A can be moved closer to each other, so that a higher overlap region between the top channel 21 and the bottom channel 31 can be achieved. The top channel 21 and/or the bottom channel 31 may have a length, such as a distance between a respective inlet and outlet of the top channel 21 and/or the bottom channel 31, being a multiple of 4.5 mm. As can be seen in Fig. 5, the first bottom inlet opening 44A and the first bottom outlet opening 46A are arranged in the membrane 4 so that they overlap with the bottom inlet port 24A and the bottom outlet port 26A respectively. In contrast there are no openings in the membrane at the locations of the top inlet port 23 and the top outlet port 25. Thus, the membrane may prevent a fluid flow through the top inlet port 23 and/or the top outlet port 25 to enter the bottom channel 31, while allowing a fluid flow through the first bottom inlet port 24A and/or the first bottom outlet port 26A to enter the bottom channel 31. The membrane may be porous and therefore some fluid flow between the top channel and the bottom channel may occur through the membrane before a confluent tissue cell layer has been formed on the membrane. Hence, preventing a fluid flow through the top inlet port 23 and/or the top outlet port 25 to enter the bottom channel 31 may herein be understood as preventing a majority of the fluid flow through the top inlet port 23 and/or the top outlet port 25 to enter the bottom channel 31. When the cell layer has been formed on the membrane the cells may control the exchange of fluids between the top channel and the bottom channel.

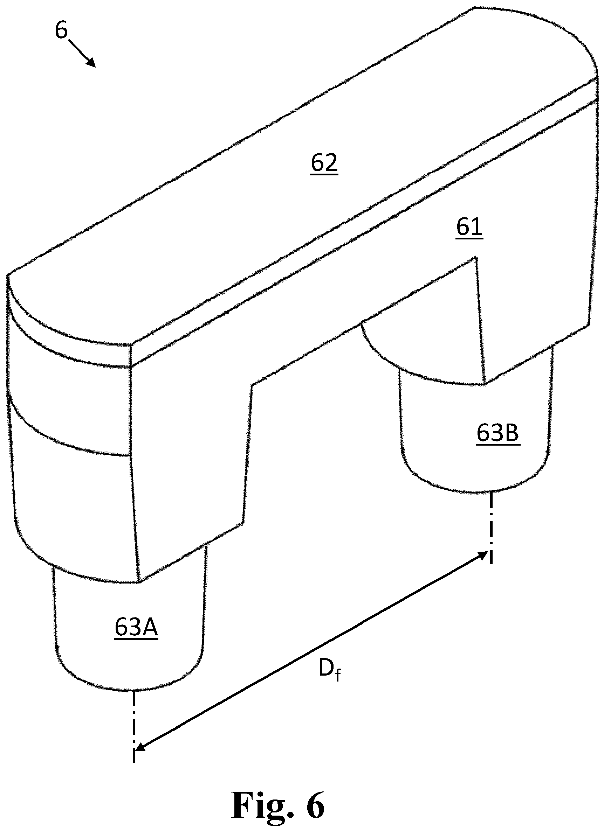

Fig. 6 illustrates a connector 6 for interconnecting the plurality of top channels 21 and/or plurality of bottom channels 31 of the microfluidic device 1 according to one or more examples of the current disclosure. The connector 6 is configured to connect an outlet port from a first top channel and/or a first bottom channel to an inlet port of a second top channel and/or a second bottom channel. By connecting the plurality of top channels and/or bottom channels using a plurality of connectors 6 a continuous fluid flow can be generated through the plurality of top channels and/or bottom channels. In one or more example connectors, the connector 6 is made from thermoplastic, such as one or more of COC, PS, PET and/or PC. Thereby, the physiological gas concentrations of the flow can be retained through the connector 6. The example connector 6 shown in Fig. 6 comprises two parts, such as a bottom connector part 61 and a top connector part 62. The bottom connector part 61 and the top connector part 62 may be bonded together to form one integral connector 6. The example bottom connector part 62 shown in Fig. 6 comprises a first fitting 63A and a second fitting 63B for interfacing with the inlets and/or outlets of the microfluidic device. The first fitting 63A and the second fitting 63B may be hollow, so that a microfluidic channel is provided in the first fitting 63A and the second fitting 63B. The first fitting 63A and the second fitting 63B may be a first and a second luer fitting, such as a first and second mini-luer fitting. In the example connector 6 of Fig. 6 the first fitting 63A and the second fitting 63B are male luer fittings. The bottom connector part 61 comprises a microfluidic channel connecting the first fitting 63A and the second fitting 63B. In the example connector 6 shown in Fig. 6, the top connector part 62 is a flat cover covering the microfluidic channel comprised in the connector 6. The top connector part 62 may be transparent to allow visual inspection of the flow through the connector 6. The distance $D_f$ between a center axis of the first fitting 63A and the second fitting 63B of the connector 6 corresponds to the distance between two parallel channels of the microfluidic device 1, such as two parallel top channels and/or two parallel bottom channels. The distance $D_f$ may be a multiple of 4.5 mm.

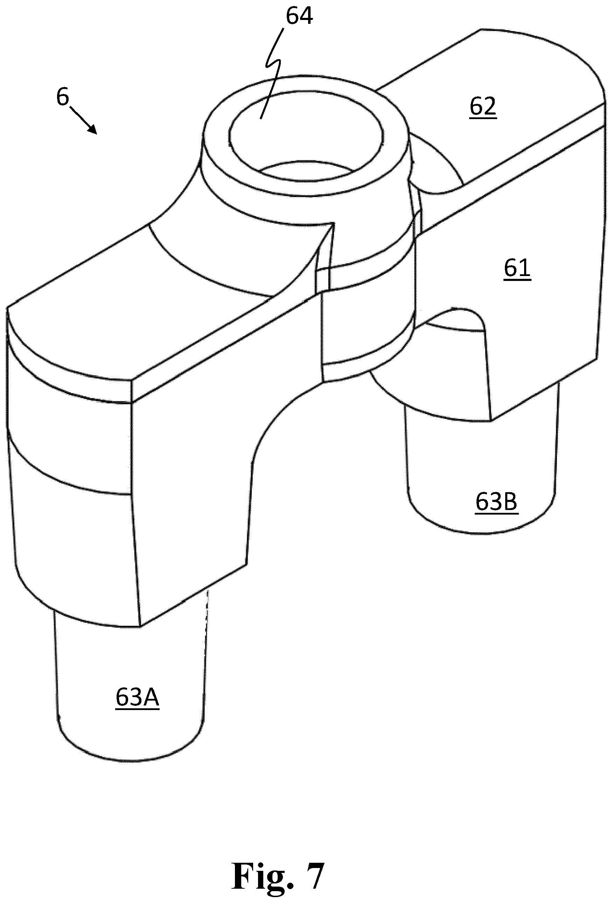

Fig. 7 illustrates a connector 6 for interconnecting the plurality of top channels 21 and/or plurality of bottom channels of the microfluidic device 1 according to one or more examples of the current disclosure. The bottom connector part 62 of the example connector shown in Fig. 7 corresponds to the bottom connector part 62 of the example connector 6 shown in Fig. 6 and comprises the first fitting 63A and the second fitting 63B for interfacing with the inlets and/or outlets of the microfluidic device 1. In the example connector 6 shown in Fig. 7, the top connector part 62 comprises a port 64, such as a sensor port, for receiving a sensor for measuring a flow, such as characteristics of the flow, through the connector 6. The port 64 is in fluid communication with the microfluidic channel of the bottom connector part 61. In one or more example microfluidic devices 1 the port 64 may be configured to receive a sensor for gas and/or analyte sensing, such as an oxygen sensor and/or a TEER sensor. The port 64 may be a luer port, such as a mini-luer port. The top connector part 62 may be transparent to allow visual inspection of the flow through the connector 6.

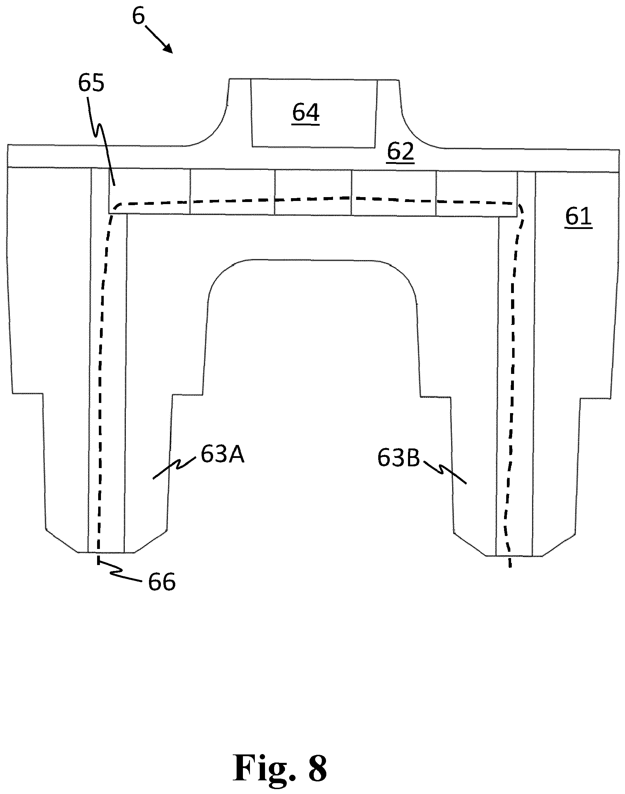

Fig. 8 is a cross-sectional view of the example connector shown in Fig. 7. The bottom connector part 61 comprises a microfluidic channel 65, connecting the first fitting 63A and the second fitting 63B. A fluid may thus flow through the microfluidic channel 64 between the first fitting 63A and the second fitting 63B along a flow path 66. The top connector part 62 may be transparent to allow visual inspection of the flow through the connector 6.

It shall be noted that the features mentioned in the embodiments described in Figs. 1-8 are not restricted to these specific embodiments. Any features relating to the microfluidic device and the components comprised therein and mentioned in relation to the microfluidic device of Figs. 4-5, such as dimensions of the microfluidic device and the channels comprised in the microfluidic device, are thus also applicable to the microfluidic device described in relation to Figs. 1-3.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

It is to be noted that the word "comprising" does not necessarily exclude the presence of other elements or steps than those listed.

It is to be noted that the words "a" or "an" preceding an element do not exclude the presence of a plurality of such elements.

Although features have been shown and described, it will be understood that they are not intended to limit the claimed disclosure, and it will be made obvious to those skilled in the art that various changes and modifications may be made without departing from the scope of the claimed disclosure. The specification and drawings are, accordingly to be regarded in an illustrative rather than restrictive sense. The claimed disclosure is intended to cover all alternatives, modifications, and equivalents.

References

1. Rubert, J. et al. Intestinal Organoids: A Tool for Modelling Diet-Microbiome-Host Interactions. Trends Endocrinol. Metab. 31, 848-858 (2020).

2. Jalili-Firoozinezhad, S. et al. A complex human gut microbiome cultured in an anaerobic intestine-on-a-chip. Nat. Biomed. Eng. 3, 520-531 (2019).

3. Huh, D. et al. Reconstituting organ-level lung functions on a chip. Science (80-.). 328, 1662-1668 (2010).

4. Herland, A. et al. Quantitative prediction of human pharmacokinetic responses to drugs via fluidically coupled vascularized organ chips. Nat. Biomed. Eng. (2020). doi:10.1038/s41551-019-0498-9

5. Shin, W., Hinojosa, C. D., Ingber, D. E. & Kim, H. J. Human Intestinal Morphogenesis Controlled by Transepithelial Morphogen Gradient and Flow-Dependent Physical Cues in a Microengineered Gut-on-a-Chip. iScience 15, 391-406 (2019).

6. Delon, L. C. et al. Hele Shaw microfluidic device: A new tool for systematic investigation into the effect of the fluid shear stress for organs-on-chips. MethodsX 7, 100980 (2020).

7. Rimsa, R., Smith, A. J., Wälti, C. & Wood, C. D. A planar surface acoustic wave micropump for closed-loop microfluidics. Appl. Phys. Lett. 111, 234102 (2017).

8. Toepke, M. W. & Beebe, D. J. PDMS absorption of small molecules and consequences in microfluidic applications. Lab Chip 6, 1484 (2006).

9. Novak, R. et al. Robotic fluidic coupling and interrogation of multiple vascularized organ chips. Nat. Biomed. Eng. (2020). doi:10.1038/s41551-019-0497-x

**Claims**

1. A microfluidic device (1) for simulating organ functions, the microfluidic device comprising a top plate (2), a bottom plate (3) and a membrane (4),

   the top plate (2) comprising a top channel (21) arranged on a first surface (22) of the top plate (2),
   the bottom plate (3) comprising a bottom channel (31) arranged on a first surface (32) of the bottom plate (3), wherein the top channel (21) is overlapping the bottom channel (31) over an entire length of the top channel (21),
   wherein the first surface (22) of the top plate (2) and the first surface (32) of the bottom plate (3) are facing each other,
   the membrane (4) being arranged between the top plate (2) and the bottom plate (3) so that the top channel (21) and the bottom channel (31) are separated by the membrane (4),
   the top plate (2) comprising a top channel inlet port (23) being in fluid communication with the top channel (21),
   wherein the top plate (2) comprises a first bottom channel inlet port (24A) and a second bottom channel inlet port (24B) both being in fluid communication with the bottom channel (31), wherein the second bottom channel inlet port (24B) is arranged further from the longitudinal center of the bottom channel (31) than the first bottom channel inlet port (24A).

**2.** The microfluidic device (1) according to claim 1, wherein the top plate (2) comprises a top channel outlet port (25) being in fluid communication with the top channel (21).

**3.** The microfluidic device (1) according to claim 1 or 2, wherein the top plate (2) comprises a first bottom channel outlet port (26A) being in fluid communication with the bottom channel (31).

**4.** The microfluidic device (1) according to claim 3, wherein the top plate (2) comprises a second bottom channel outlet port (26A) being in fluid communication with the bottom channel (31), wherein the second bottom channel outlet port (26B) is arranged further from the longitudinal center of the bottom channel (31) than the first bottom channel outlet port (26A).

**5.** The microfluidic device (1) according to any one of the previous claims, wherein the top channel inlet port (23), the first bottom channel inlet port (24A), the second bottom channel inlet port (24B), the top channel outlet port (25), the first bottom channel outlet port (26A) and/or the second bottom channel outlet port (26B) are arranged perpendicular to the second surface (28) of the top plate (2).

**6.** The microfluidic device (1) according to any one of the previous claims, wherein the top plate (2) comprises a bottom channel sensor port (27A) for receiving a sensor for monitoring the bottom channel (31).

**7.** The microfluidic device (1) according to any one of the previous claims, wherein the top plate (2) comprises a top channel sensor port (27B) for receiving a sensor for monitoring the top channel (21).

**8.** The microfluidic device (1) according to claim 6 and 7, wherein the top channel sensor port (27B) and/or the bottom sensor port (27A) comprises a raised portion (24) protruding from a second surface (28) of the top plate (2), wherein the top sensor port protrudes further from the second surface than the bottom sensor port.

**9.** The microfluidic device (1) according to any one of the previous claims, wherein the top channel (21) and the bottom channel (31) extend in a longitudinal direction of the top plate (2) and the bottom plate (3) respectively.

**10.** The microfluidic device (1) according to any one of the previous claims, wherein the top plate (2) and/or the bottom plate (3) is made out of one or more of cyclic olefin copolymer, COC, polystyrene, PS, and polycarbonate, PC.

**11.** The microfluidic device (1) according to any one of the previous claims, wherein the top plate (2) and/or the bottom plate (3) are injection molded.

**12.** The microfluidic device (1) according to any one of the previous claims, wherein the membrane (4) is made out of one or more of cyclic olefin copolymer, COC, polyethylene terephthalate, PET, and polycarbonate, PC.

**13.** The microfluidic device (1) according to any one of the previous claims, wherein the top plate (2) and/or the bottom plate (3) and/or the membrane (4) are bonded together using ultrasonic welding.

**14.** The microfluidic device (1) according to any one of the previous claims, wherein the top channel (21) has a height in the range of 0.2 to 2 mm.

**15.** The microfluidic device (1) according to any one of the previous claims, wherein the bottom channel (31) has a height in the range of 0.1 to 0.5 mm.

**16.** The microfluidic device (1) according to any one of the previous claims, wherein the top channel (21) and/or the bottom channel (31) has a width in the range of 0.25 to 2 mm.

**17.** The microfluidic device (1) according to any one of the previous claims, wherein the top channel (21) and/or the bottom channel (31) has a length being a multiple of 4.5 mm.

**18.** The microfluidic device (1) according to any one of the previous claims, wherein the first bottom channel inlet port (24A), the second bottom channel inlet port (24B) and/or the top channel inlet port (23) are arranged at a distance being a multiple of 4.5 mm from each other.

**19.** The microfluidic device (1) according to any one of the previous claims, wherein the top plate (2) comprises a plurality of top channels (21) and the bottom plate (3) comprises a plurality of bottom channels (31).

**20.** The microfluidic device (1) according to claim 19, wherein the plurality of top channels (21) and the plurality of bottom channels (31) are arranged in parallel to each other.

**21.** The microfluidic device (1) according to claim 19 or 20, wherein the microfluidic device (1) comprises a connector (6) for interconnecting the plurality of top channels (21) and/or bottom channels (31).

**22.** The microfluidic device (1) according to claim 21, wherein the connector (6) comprises a sensor port (64) for receiving a sensor for measuring a flow through the connector (6).

23. The microfluidic device (1) according to any one of the claims 19 to 22, wherein the plurality of top channels (21) and/or bottom channels (31) have different height, length and/or width.

24. The microfluidic device (1) according to any one of the claims 19 to 22, wherein the plurality of top channels (21) and/or bottom channels (31) have the same height, length and/or width.

25. The microfluidic device (1) according to any one of the previous claims, wherein the top plate (2) and/or the bottom plate (3) comprises a first tensioning element (51) for tensioning the membrane (4) during assembly of the microfluidic device (1).

26. The microfluidic device (1) according to claim 25, wherein the top plate (2) and/or the bottom plate (3) comprises a second tensioning element (52), the first tensioning element (51) and the second tensioning element (52) being arranged on opposite sides of the top channel (31) and/or the bottom channel (31).

**Fig. 1**

**Fig. 2**

Fig. 3

Fig. 4

**Fig. 5**

EP 4 198 119 A1

**Fig. 6**

**Fig. 7**

6

65

64

62

61

63A

63B

66

**Fig. 8**

**EUROPEAN SEARCH REPORT**

Application Number

EP 21 21 5336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/118799 A2 (HARVARD COLLEGE [US]; INGBER DONALD E [US]; KIM HYUN JUNG [US]) 7 September 2012 (2012-09-07) | 1,9-16 | INV. C12M3/06 |
| Y | * paragraphs [0003], [0005], [0006], [0013], [0014], [0018], [0024], [0028], [0029], [0079], [0082], | 1-7, 9-21, 23-26 | |
| A | [0090]; figures 3A-D * | 8,22 | |
| | ----- | | |
| X | WO 2019/195344 A1 (HARVARD COLLEGE [US]) 10 October 2019 (2019-10-10) | 1,6,7,9 | |
| Y | * paragraphs [0012], [0026], [0271], [0275]; figure 1B * | 1-7, 9-21, 23-26 | |
| A | | 8,22 | |
| | ----- | | |
| X | ASHAMMAKHI NUREDDIN ET AL: "Gut-on-a-chip: Current progress and future opportunities", BIOMATERIALS, ELSEVIER, AMSTERDAM, NL, vol. 255, 14 June 2020 (2020-06-14), XP086230849, ISSN: 0142-9612, DOI: 10.1016/J.BIOMATERIALS.2020.120196 [retrieved on 2020-06-14] | 1,12 | |
| Y | * page 4, column 1, paragraphs 1,2 * * page 2, column 1, paragraph 2.1 * * page 3, column 1, paragraph 2.2 * | 1-7, 9-21, 23-26 | TECHNICAL FIELDS SEARCHED (IPC) C12M |
| A | * page 4, column 1, paragraphs 1,2 – column 2, paragraph 3 * * page 6, paragraphs 2.5,3 * * page 8, column 1, paragraph 1-2 * | 8,22 | |
| | ----- | | |
| Y | US 2014/306371 A1 (GUENTHER AXEL [CA] ET AL) 16 October 2014 (2014-10-16) * paragraph [0129]; figures * | 1-7, 9-21, 23-26 | |
| | ----- -/-- | | |

The present search report has been drawn up for all claims

| Place of search | Date of completion of the search | Examiner |
|---|---|---|
| Berlin | 8 June 2022 | Böhm, Ingo |

EPO FORM 1503 03.82 (P04C01)

page 1 of 2

Europäisches
Patentamt

European
Patent Office

Office européen
des brevets

# EUROPEAN SEARCH REPORT

Application Number

EP 21 21 5336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/009307 A2 (CHILDRENS MEDICAL CENTER [US]; INGBER DONALD E [US]; HUH DONGEUN [US]) 21 January 2010 (2010-01-21) * paragraphs [0019] - [0021], [0066], [0165], [0166]; figure 7A * ----- | 1-7, 9-21, 23-26 | |
| Y | US 2015/247112 A1 (ORR DAVID E [US] ET AL) 3 September 2015 (2015-09-03) * paragraphs [0055], [0059], [0066] * ----- | 1-7, 9-21, 23-26 | |
| A | US 2016/220997 A1 (MESCHER MARK JOSEPH [US] ET AL) 4 August 2016 (2016-08-04) * paragraphs [0003], [0005]; figures * ----- | 1-26 | |
| A | WO 2019/178251 A1 (EMULATE INC [US]) 19 September 2019 (2019-09-19) * pages 1-4 * * page 67, paragraph 3 * * page 68, paragraph 3 * ----- | 1 | |
| A | WO 2018/052953 A1 (HARVARD COLLEGE [US]) 22 March 2018 (2018-03-22) * paragraphs [0078] - [0109]; figures * ----- | 1 | TECHNICAL FIELDS SEARCHED (IPC) |
| A | US 2020/224136 A1 (KASENDRA MAGDALENA [US] ET AL) 16 July 2020 (2020-07-16) * paragraphs [0002], [0008] - [0018] * ----- | 1 | |

The present search report has been drawn up for all claims

| Place of search | Date of completion of the search | Examiner |
|---|---|---|
| Berlin | 8 June 2022 | Böhm, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
### ON EUROPEAN PATENT APPLICATION NO.

**EP 21 21 5336**

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2022

| Patent document cited in search report | Publication date | Patent family member(s) | | Publication date |
|---|---|---|---|---|
| WO 2012118799 A2 | 07-09-2012 | AU | 2012223526 A1 | 12-09-2013 |
| | | AU | 2017202616 A1 | 11-05-2017 |
| | | AU | 2019202169 A1 | 18-04-2019 |
| | | AU | 2019268194 A1 | 12-12-2019 |
| | | CA | 2828110 A1 | 07-09-2012 |
| | | CN | 103502426 A | 08-01-2014 |
| | | DK | 2681306 T3 | 23-04-2019 |
| | | EP | 2681306 A2 | 08-01-2014 |
| | | EP | 3498818 A1 | 19-06-2019 |
| | | JP | 6122388 B2 | 26-04-2017 |
| | | JP | 6457007 B2 | 23-01-2019 |
| | | JP | 2014506801 A | 20-03-2014 |
| | | JP | 2017136082 A | 10-08-2017 |
| | | KR | 20140040704 A | 03-04-2014 |
| | | KR | 20190028810 A | 19-03-2019 |
| | | KR | 20200060543 A | 29-05-2020 |
| | | RU | 2013143537 A | 10-04-2015 |
| | | SG | 192931 A1 | 30-09-2013 |
| | | SG | 10201601434R A | 30-03-2016 |
| | | US | 2014038279 A1 | 06-02-2014 |
| | | US | 2017101628 A1 | 13-04-2017 |
| | | US | 2020087608 A1 | 19-03-2020 |
| | | WO | 2012118799 A2 | 07-09-2012 |
| WO 2019195344 A1 | 10-10-2019 | AU | 2019247650 A1 | 29-10-2020 |
| | | CA | 3095749 A1 | 10-10-2019 |
| | | GB | 2587142 A | 17-03-2021 |
| | | US | 2021079356 A1 | 18-03-2021 |
| | | WO | 2019195344 A1 | 10-10-2019 |
| US 2014306371 A1 | 16-10-2014 | CA | 2856063 A1 | 30-05-2013 |
| | | EP | 2782866 A1 | 01-10-2014 |
| | | US | 2014306371 A1 | 16-10-2014 |
| | | WO | 2013075248 A1 | 30-05-2013 |
| WO 2010009307 A2 | 21-01-2010 | AU | 2009270821 A1 | 21-01-2010 |
| | | CA | 2730928 A1 | 21-01-2010 |
| | | CN | 102124096 A | 13-07-2011 |
| | | CN | 104328050 A | 04-02-2015 |
| | | CN | 107988072 A | 04-05-2018 |
| | | DK | 2313487 T3 | 18-06-2018 |
| | | DK | 3404093 T3 | 16-03-2020 |
| | | EP | 2313487 A2 | 27-04-2011 |
| | | EP | 3404093 A1 | 21-11-2018 |
| | | EP | 3693454 A1 | 12-08-2020 |
| | | EP | 3778858 A1 | 17-02-2021 |

EPO FORM P0459

For more details about this annex : see Official Journal of the European Patent Office, No. 12/82

**page 1 of 3**

## ANNEX TO THE EUROPEAN SEARCH REPORT
## ON EUROPEAN PATENT APPLICATION NO.

EP 21 21 5336

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2022

| Patent document cited in search report | Publication date | Patent family member(s) | Publication date |
|---|---|---|---|
| | | ES 2672201 T3 | 13-06-2018 |
| | | HK 1159158 A1 | 27-07-2012 |
| | | HK 1206778 A1 | 15-01-2016 |
| | | JP 5415538 B2 | 12-02-2014 |
| | | JP 5815643 B2 | 17-11-2015 |
| | | JP 6254127 B2 | 27-12-2017 |
| | | JP 6698619 B2 | 27-05-2020 |
| | | JP 2011528232 A | 17-11-2011 |
| | | JP 2014073125 A | 24-04-2014 |
| | | JP 2016000051 A | 07-01-2016 |
| | | JP 2018038425 A | 15-03-2018 |
| | | JP 2019213562 A | 19-12-2019 |
| | | JP 2022009363 A | 14-01-2022 |
| | | KR 20110044226 A | 28-04-2011 |
| | | KR 20170124616 A | 10-11-2017 |
| | | KR 20190060894 A | 03-06-2019 |
| | | KR 20200092436 A | 03-08-2020 |
| | | KR 20220016289 A | 08-02-2022 |
| | | US 2011250585 A1 | 13-10-2011 |
| | | US 2014093905 A1 | 03-04-2014 |
| | | US 2014093906 A1 | 03-04-2014 |
| | | US 2014147880 A1 | 29-05-2014 |
| | | US 2016046896 A1 | 18-02-2016 |
| | | US 2016046897 A1 | 18-02-2016 |
| | | US 2017349871 A1 | 07-12-2017 |
| | | US 2019352590 A1 | 21-11-2019 |
| | | WO 2010009307 A2 | 21-01-2010 |
| US 2015247112 A1 03-09-2015 | | EP 3114207 A1 | 11-01-2017 |
| | | US 2015247112 A1 | 03-09-2015 |
| | | WO 2015134550 A1 | 11-09-2015 |
| US 2016220997 A1 04-08-2016 | | AU 2016215169 A1 | 10-08-2017 |
| | | CA 2975182 A1 | 11-08-2016 |
| | | CN 107454862 A | 08-12-2017 |
| | | EP 3254008 A1 | 13-12-2017 |
| | | JP 2018516065 A | 21-06-2018 |
| | | US 2016220997 A1 | 04-08-2016 |
| | | WO 2016127009 A1 | 11-08-2016 |
| WO 2019178251 A1 19-09-2019 | | GB 2586724 A | 03-03-2021 |
| | | US 2021054323 A1 | 25-02-2021 |
| | | WO 2019178251 A1 | 19-09-2019 |
| WO 2018052953 A1 22-03-2018 | | AU 2017326179 A1 | 28-03-2019 |
| | | CA 3036378 A1 | 22-03-2018 |

EPO FORM P0459

For more details about this annex : see Official Journal of the European Patent Office, No. 12/82

page 2 of 3

**ANNEX TO THE EUROPEAN SEARCH REPORT**
**ON EUROPEAN PATENT APPLICATION NO.**                EP 21 21 5336

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2022

| Patent document cited in search report | Publication date | Patent family member(s) | | Publication date |
|---|---|---|---|---|
| | | EP | 3512935 A1 | 24-07-2019 |
| | | GB | 2570593 A | 31-07-2019 |
| | | US | 2020231938 A1 | 23-07-2020 |
| | | US | 2022145266 A1 | 12-05-2022 |
| | | WO | 2018052953 A1 | 22-03-2018 |
| US 2020224136 A1 | 16-07-2020 | NONE | | |

EPO FORM P0459

For more details about this annex : see Official Journal of the European Patent Office, No. 12/82

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RUBERT, J et al.** Intestinal Organoids: A Tool for Modelling Diet-Microbiome-Host Interactions. *Trends Endocrinol. Metab.,* 2020, vol. 31, 848-858
- **JALILI-FIROOZINEZHAD, S. et al.** A complex human gut microbiome cultured in an anaerobic intestine-on-a-chip. *Nat. Biomed. Eng.,* 2019, vol. 3, 520-531
- **HUH, D. et al.** Reconstituting organ-level lung functions on a chip. *Science,* 2010, vol. 328, 1662-1668
- **HERLAND, A. et al.** Quantitative prediction of human pharmacokinetic responses to drugs via fluidically coupled vascularized organ chips. *Nat. Biomed. Eng.,* 2020

- **SHIN, W. ; HINOJOSA, C. D. ; INGBER, D. E. ; KIM, H. J.** Human Intestinal Morphogenesis Controlled by Transepithelial Morphogen Gradient and Flow-Dependent Physical Cues in a Microengineered Gut-on-a-Chip. *iScience,* 2019, vol. 15, 391-406
- **DELON, L. C. et al.** Hele Shaw microfluidic device: A new tool for systematic investigation into the effect of the fluid shear stress for organs-on-chips. *MethodsX,* 2020, vol. 7, 100980
- **RIMSA, R. ; SMITH, A. J. ; WÄLTI, C. ; WOOD, C. D.** A planar surface acoustic wave micropump for closed-loop microfluidics. *Appl. Phys. Lett.,* 2017, vol. 111, 234102
- **TOEPKE, M. W. ; BEEBE, D. J.** PDMS absorption of small molecules and consequences in microfluidic applications. *Lab Chip,* 2006, vol. 6, 1484
- **NOVAK, R. et al.** Robotic fluidic coupling and interrogation of multiple vascularized organ chips. *Nat. Biomed. Eng.,* 2020